# EUROPEAN PATENT SPECIFICATION

(11) **EP 0 565 251 B1**
(45) Date of publication and mention of the grant of the patent: **20.05.1998**
(21) Application number: 93302066.1
(22) Date of filing: 18.03.1993
(51) Int. Cl.: A61M 29/00, A61F 2/06

(54) **Vascular stent**
Einrichtung zur Aufweitung von Blutgefässen
Stent vasculaire

(30) Priority: 25.03.1992 US 858304; 24.04.1992 US 874347; 10.09.1992 US 943000
(43) Date of publication of application: 13.10.1993
(73) Proprietor: Cook Incorporated, Bloomington IN 47402-0489 (US)
(72) Inventor: Fontaine, Arthur B., Fresno, California (US); Dake, Michael D., Stanford, California (US)
(74) Representative: Johnston, Kenneth Graham

(56) References cited:
- US-A- 4 733 665
- US-A- 4 800 882

## Description

The present invention generally relates to vascular stents.

A stent, generally speaking, is a device that can be placed within the lumen, or interior space, of a tubular structure for supporting and assuring patency of a contracted, but otherwise intact, lumen. (Patency the state of being freely open, is particularly important in the field of angioplasty, which is concerned with the reconstruction of blood vessels.) Stents are used, for example, for holding blood vessels open or for back tacking intimal flaps inside vessels after angioplasty. More generally, however, stents can be used inside the lumina of any physiological conduit including arteries, veins, vessels, the biliary tree, the urinary tract, the alimentary tract, the tracheobronchial tree, the genitourinary system, and the cerebral aqueduct. Furthermore, stents can be used inside lumina of animals other than humans.

In the field of angioplasty, the most common angioplasty surgical procedure is percutaneous transluminal coronary angioplasty, or "PTCA", which is employed for enlarging narrowed arteries near the heart. In a PTCA procedure, a balloon-tip catheter is maneuvered into position in a narrowed artery where the balloon is expanded in order to dilate this area of narrowing. After the arterial lumen is dilated, the balloon at the catheter tip is deflated and the catheter is removed from the enlarged artery. A vascular stent can be used to dilate an artery after a suboptimal PTCA dilation.

In practice, the above-described conventional PTCA procedure has several shortcomings. One drawback is that approximately one-third of all PTCA patients suffer from restenosis, a chronic regrowth of obstructive tissue that narrows the lumen. Typically, restenosis occurs within six months following an angioplasty procedure. Since a majority of these restenosis patients also display symptoms of deteriorating cardiac status, they frequently must undergo additional PTCA procedures or more risky coronary artery bypass graft surgery. Unfortunately, those patients who undergo repeated PTCA procedures tend to restenose at an even higher rate than first-time PTCA patients.

A second, and sometimes fatal, complication of coronary angioplasty is the abrupt re-closure of a previously dilated section of a vessel. There are many different factors that are thought to contribute to abrupt re-closure after PTCA including obstructive flaps of disrupted wall tissue, vessel wall spasms with luminal contraction, and thrombus formation at the site of dilation. Vascular stents can be used like a scaffold to mechanically bridge areas of narrowing (flaps or thrombus) and oppose spasms, and therefore, maintain artery patency.

Many of the factors responsible for abrupt closure (post balloon inflation) may also influence the development of restenosis, and therefore, long term patency. In this regard, vascular stents, by virtue of their ability to limit elastic recoil of the vessel wall and to eliminate the negative physical consequences of PTCA (including obstructing intimal flaps and dissection) may be useful in limiting restenosis.

Therefore, there are two potential benefits of vascular stents in the treatment of vascular disease: 1) prevention of abrupt arterial closure, and 2) prevention of restenosis.

U.S. Patent 4,733,665 discloses a vascular stent with wires overlapping one another, and with spot welded intersections.

According to the present invention there is provided a stent as defined in claim 1 and a method as defined in claim 7.

Generally speaking, the present invention provides a vascular stent for reducing hemodynamic disturbances caused by angioplasty and the stent itself. In a preferred embodiment, the stent is formed from a single filament of low memory bio-compatible material having a series of U-shaped bends. The filament is wrapped around a mandril in a circular fashion in order to align opposing curved portions of each bend which are then connected. The stent therefore provides a maximum amount of structural support for the lumen while minimizing the level of hemodynamic disturbance inside the lumen.

The tubular stent shown in the embodiments of the invention is a co-planar structure as opposed to a woven or knitted structure.

The present inventor has found that vascular stents require substantial flexibility in their unexpanded state in order to allow them to bend and conform to the tortuous shape of the vessel through which they are inserted. This need for flexibility during insertion is especially important for older patients since their blood vessels tend to be more tortuous and less flexible than those of younger patients. The present inventor has also found that, vascular stents should be rigid and have a high hoop strength in their expanded state. Although the reasons for the success of rigid stents are not entirely clear, it has been suggested that rigid stents are less likely to pulsate inside vessels, and therefore, they are less likely to rub against the vessel intima once they are in place.

### Brief Description of the Drawings

Figure 1 shows a filament shaped into a compressed planar wave used to make the nearly sinusoidal waveform of Figure 2;
Figure 2 shows the planar wave of Figure 1 expanded along its longitudinal centerline to form a nearly sinusoidal waveform used in making a stent;
Figure 3 shows an alternative waveform that can also be used in making a stent;
Figure 4 shows another alternative waveform that can be used in making a stent;
Figure 5 shows the waveform of Figure 3 spirally wrapped around a round mandril;
Figure 6 shows a connection for the end of the filament after the waveform of Figure 3 is completely wrapped around the mandril;
Figure 7 shows a preferred alternative waveform that can be used in making a stent;
Figure 8 shows the relative positions of the U-shaft bends in each component section of the preferred alternative waveform of Figure 12;
Figure 9 shows the preferred alternative waveform of Figure 7 being wrapped around a cylindrical mandril;
Figure 10 shows in an expanded state a side elevation of a stent formed from the preferred alternative waveform of Figure 7 by wrapping it around a mandril in a circular fashion in order to align the curved portion of each bend;
Figure 11 shows an opposite side elevation of the stent in Figure 10;
Figure 12 shows an end view of the stents in Figures 10 and 11;
Figure 13 shows a stent mounted on a balloon-tip catheter ready for insertion into a lumen;
Figure 14 shows a stent being used with a graft to repair a pseudo-aneurysm in the common femoral artery;
Figure 15 shows two stents being used with a graft to bypass an occlusion in the femoral-popliteal artery;
Figure 16 shows a stent being used with a graft to repair an aorto-iliac aneurysm;
Figure 17 is a schematic illustration of a planar waveform which is used to form the stent;
Figure 18 illustrates the waveform of Figure 17 being wrapped around a mandril;
Figure 19 illustrates an alternative embodiment of the waveform of Figure 17 being wrapped around a mandril;
Figure 20 shows the arrangement of the waves around the circumference of the mandril when the stent is formed in its unexpanded state as in Figure 18;
Figure 21 shows the arrangement of the waves around the circumference of the mandril when the stent is formed in its unexpanded state as in Figure 19;
Figure 22 is an enlargement of one of the cells in the stent of Figures 20 and 21 when the stent is in an expanded state;
Figure 23 shows a stent being used with a graft;
Figure 24 is a side elevational view of a stent according to the other preferred embodiment in compressed condition;
Figure 25 is a side elevational view of the stent of Figure 24 in expanded condition;
Figure 26 is an end view of the stent of Figure 25;
Figure 27 is a cross-sectional view which is taken along the plane of the line 4-4 in Figure 25 for viewing in the direction of the arrows;
Figure 28 is an enlarged cross-sectional detail, taken along the plane of the line 5-5 in Figure 26 for viewing in the direction of the arrows;
Figures 29A and 29B are views that correspond in orientation to Figure 4 and which schematically show the stent of Figure 1 embedded in the lumen of a blood vessel;
Figure 30 is a schematic illustration of a planar waveform of a continuous wire which is used to form the stent of Figures 24 and 25; and
Figures 31A and 31B are illustration of the continuous waveform of Figure 27 wrapped around the circumference of a mandrel for forming the stent in its compressed condition.

The stent is preferably formed from a continuous wire. The term "wire", as used here, should not be construed as limited to just metallic materials. In fact, the stent may be formed from any type of filament. The stent may also be made from groups of filaments or fibers which are wound or braided together in order to form a continuous filament. Also, several distinct filaments may also be attached together by any conventional means such as butt-welding.

To prevent the stent from recoiling to its unexpanded state after it has been implanted, the stent is preferably made from a "low memory" material that does not try to resume its original shape after it is deformed. Alternatively, the size of the wire can be chosen so that when the stent is expanded, the wire is stressed but not beyond the ultimate stress at which the material cracks or breaks. Both the unformed wire and the unexpanded stent may be annealed in order to reduce the stresses which are created in the wire during the stent formation process.

The stent material is preferably radio-opaque so that the location of the stent can be verified through fluoroscopic examination. The stent should also be made from a biocompatible (e.g. stainless steel) and/or bioabsorbable (e.g. Vicryl) material with a smooth surface for minimizing the stent's effect on surrounding tissue and bodily fluids such as blood. The stent may also be coated with antithromblytic or anticoagulatory agents such as Dextran, Heperin, t-PA, polytetrafluoroethylene, or ultra low-temperature isotropic carbon.

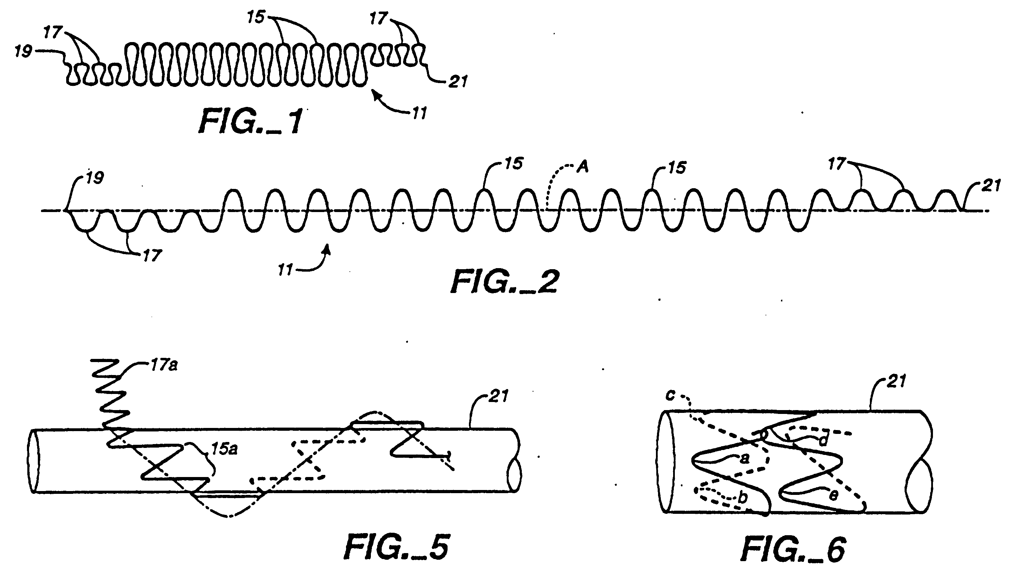

Figure 1 shows a filament 11 formed in a compressed planar waveform. Preferably, the filament 11 is made from 0.013 to 0.05 cms (0.005 - 0.020 inch) diameter stainless steel wire; however, it can be made from materials such as titanium, tantalum, gold, copper and copper alloys, combinations of these materials, or any other biologically-compatible materials with a low shape-memory level. (In the present context, a low shape-memory level implies that the stent will not contract to its compressed shape after it is inserted and internally expanded in a lumen). The filament 11 can also be formed from several separate strands which are wrapped or woven together.

The compressed waveform pattern in Figure 1 is preferably formed generally in the shape of a compressed sinusoid, but can have any wave-like pattern. In the drawing, it should be noted that the waveforms at the ends 19 and 21 of the wire having smaller amplitudes than the waveforms 15 in the middle of the wire. The drawing shows, for example, four reduced amplitude peaks 17 at each of the ends 19 and 21, respectively. Preferably, the heights of the reduced amplitude waveforms are one-half to two-thirds of the heights of the larger waveforms.

In Figure 2, the compressed waveforms of Figure 1 are expanded along their longitudinal centerline into a nearly sinusoidal waveform by stretching the compressed waveforms from their ends. (The broken line shows the longitudinal centerline of the expanded waveforms). At both ends 19 and 21, the longitudinal centerline of the smaller waveforms is displaced from the longitudinal centerline of the waveforms near the middle of the wire. At one end 19, for instance, the centerline of the smaller waveforms 17 is displaced below the broken line; at the end 21, by way of contrast, the centerline of the smaller waveforms is displaced above the broken line.

In practice, the above-described expanded waveforms preferably have a period of about eight millimeters. The larger waveforms 15 preferably have a peak-to-peak amplitude of eight millimeters while the smaller waveforms 17 are one-half to two-thirds the height of the larger waveforms. However, other sizes may be used. Although all of the waveforms normally have the same period, they are not necessarily sinusoidal, regular, repeating, or continuous.

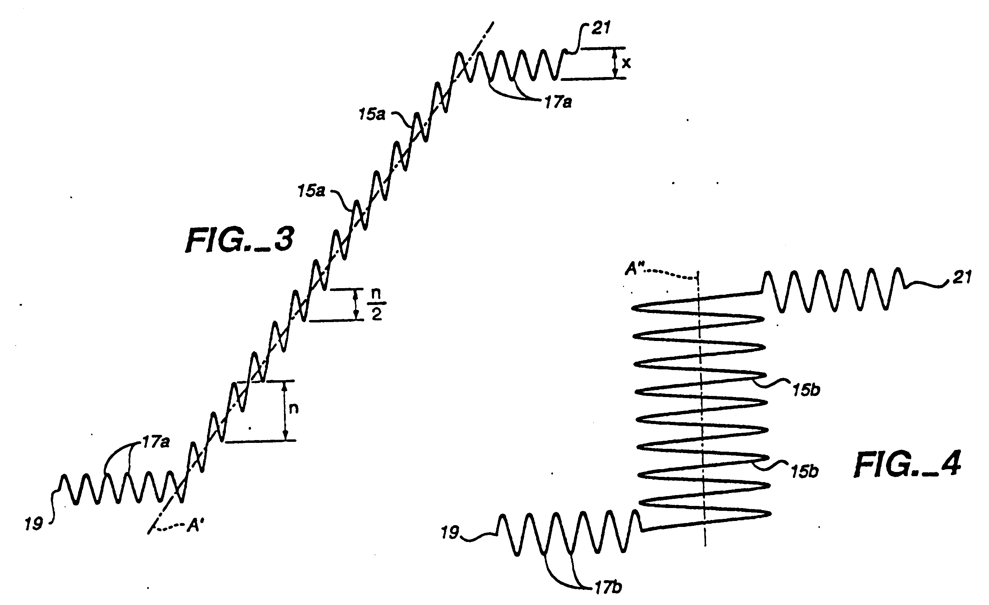

Figures 3 and 4 show the expanded state of two alternative waveforms that can be used to form the above-described stent. The period of each waveform in the waveform of Figure 3 is preferably one-half of the peak to peak amplitude of the waveform. In Figure 3, the longitudinal centerlines of the small waveforms 17a at the ends of the device are approximately parallel to each other, but the centerline of the large waveforms 15a is inclined relative to the longitudinal centerlines of the smaller waveforms, preferably at an inclination angle of approximately 45°. In Figure 4, the waveform is similar to that of Figure 3 except that the centerline of the larger waveforms 15b is perpendicular to the centerline of the smaller waveforms 17b; in other words, the inclination angle of the larger waveforms is approximately 90°.

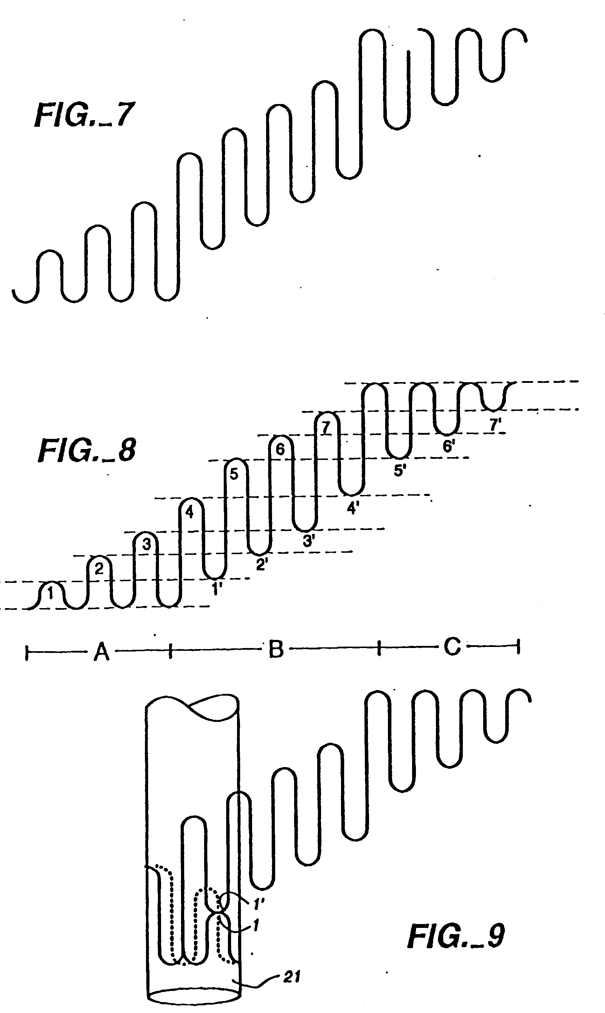

Figure 5 shows the expanded waveform of Figures 3 formed into a stent by wrapping it, in a spiral, around a mandril 21. Similar waveforms could also be used. For instance, if the waveform of Figure 4 were used the longitudinal centerline of the large waveforms would remain parallel to the centerline of the mandril and the peaks of the waveforms would be wrapped around the mandril, perpendicular to the centerline of the mandril.

As shown in Figure 5, the centerline of the large waveforms 15a is arranged to spiral along the length of the mandril 31. One side of each of the larger waveforms 15a is arranged approximately parallel to the longitudinal axis of the mandril 31, and the remaining sections of each of the waveforms are arranged at a small angle to the longitudinal axis of the mandril. (In the drawing, the "small" angle has been greatly exaggerated for purposes of illustration). It will be appreciated that the shown arrangement allows the stent to be wound in a very tight spiral.

By forming the above-described stent as a tight spiral on a mandril, the stent expands primarily in the radial direction, with relatively slight movement at the ends, as it is expanded internally in a lumen. Even greater radial expansion might be achieved by the wrapping the waveform as a circle around the mandril. However, such a radially-wrapped configuration would use an excessive amount of filament per unit surface area to support the lumen, especially where the filaments were allowed to overlap.

In Figure 6, each of the last three smaller waveforms 17a (from Figure 5) at the end of the stent is wrapped with its longitudinal centerline around the circumference of the mandril. It should be noted that the peaks of the last three smaller waveforms (indicated in Figure 6 by the letters "a", "b" and "c" respectively) are approximately the same distance from the edge of the mandril, and the fourth peak "d" is slightly further away from the end of the mandril. Also, the end of the stent near peak "a" is connected to the apex of peak "d", the result of this connection is that peaks "a", "b" and "c" are substantially equally spaced around the circumference of the mandril and are all at the approximately same distance from the end of the mandril.

In practice, the connection between the loop and the filament is slidable along the filament 11, thereby allowing for radial expansion. Although this connection can be easily made using a loop as shown, it can also be made by, for example, using a bracket. The connector could also be made by brazing, welding, or gluing the end to the filament.

When the above-described stent is wound around a mandril in the shape of a tight spiral, the non-expanded form of the stent provides a prole that is lower than conventional stents, and the "tines" of the non-expanded stent are almost parallel and packed closely together. This is important because such stent can be accommodated through a smaller incision and, therefore, reduces blood loss during surgery. Furthermore, such a stent can provide an expansion ratio of about 10:1, enabling it to be used in large arteries.

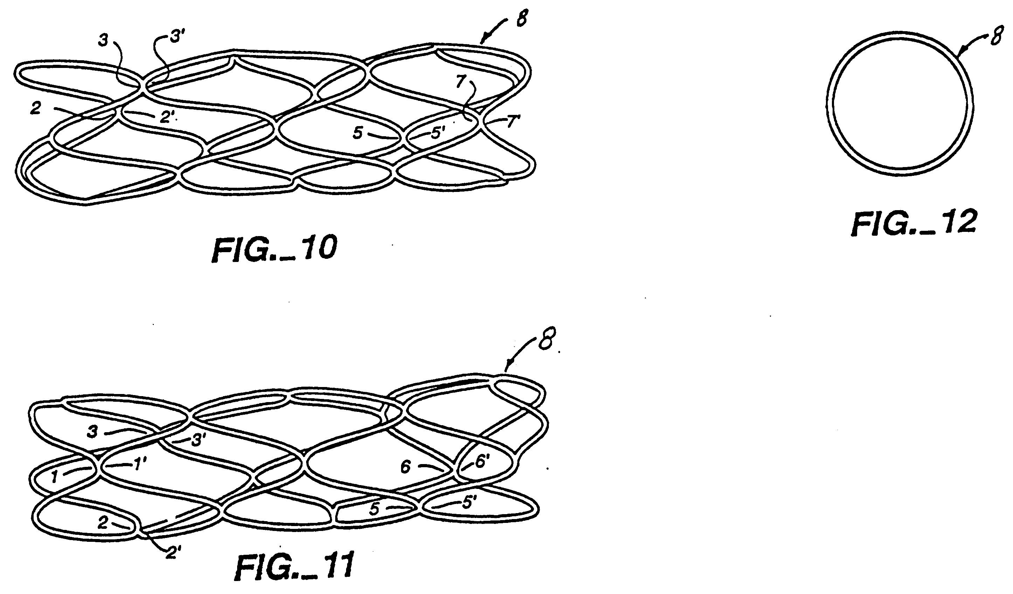

As shown in Figure 12, the connections at the ends of the filament 11 create a circular hoop near each end of the stent with no sharp edges, or point, protruding from the perimeter to project into a lumen or to catch on the balloon or plaque inside of a vessel. Also, because the centerline of the smaller waveforms is arranged along the circumference of the stent, the end hoops allow the stent to fit snugly inside the lumen and prevent migration. In other words, in this arrangement, the hoops expand radially to lock the expanded stent in place in a lumen while permitting only limited longitudinal expansion.

Figure 7 shows a preferred alternative waveform which can be used in making a stent. The waveform of Figure 7 is formed from a series of U-shaped bends having substantially straight legs on each side of the curved portion of each "U". The legs are preferably parallel; but they may also be formed at angles to each other. The curved portions are preferably semi-circular; however, other shapes of curves can be used to connect the straight legs in each bend. The curved portions may have the same or different sizes. It is also preferred that the curved portions are connected to the straight portions at the tangent of each curve in order to prevent any discontinuities in the length of the filament.

Figure 8 shows the relative positions of the U-shaped bends for each component section A, B, C of the preferred alternative waveform of Figure 7. Sections A and C of the waveform are upside down mirror images of each other. The broken lines in Figure 8 are reference lines which are preferably equally spaced and parallel. However, it is also possible to form the stent so that the top and bottom reference lines are parallel to each other but not equally spaced from or parallel to the other reference lines.

Defining the distance between the reference lines as one unit of measurement, then each of the U-shaped bends in end sections A and C each have a different length. For example, U-shaped bend 1 is one unit long while U-shaped bend 3 is three units long. Similarly, U-shaped bend 7' is one unit long while U-shaped bend 5' is three units long. In contrast, each of the waveforms in section B has one long leg which is four units long and one short leg which is three units long. For example, the left leg of U-shaped bend 5 is four units long while the right leg is three units long as measured between the reference lines. Each of the curved portions, except for the ends of the filament, are preferably semicircular with a diameter of one unit. The curved portion at each end of the filament is preferably one half of the semicircular arc. However, other shapes and proportions may also be used to appropriately size the stent.

As shown in Figure 9, the stent is formed by wrapping the waveform of Figure 7 around a mandril which is preferably cylindrical. However, mandrils with other shapes could also be use The waveform is preferably wrapped around the mandril so that the legs of each U-shaped bend are parallel to the axis of the mandril. In this configuration, a single wire may be formed into an extremely rigid tubular structure with very little material to disturb flow inside the lumen. However, the waveform might also be wrapped around the mandril in a slightly spiral manner. Once the waveform is wrapped around the mandril, the outside edge of curves on the same reference line will be arranged back-to-back adjacent to each other. For example, the outside edge of curve 1 will be back-to-back with the outside edge of curve 1'. Similarly, the outside edge of curve 7 will be adjacent to curve 7'. The outside edges of these U-shaped bends can then be fastened together by any conventional means such as welding, brazing, soldering, or gluing.

Figures 10, 11, and 12 illustrate the stent which is formed by wrapping the waveform of Figure 8 around a circular mandril with the reference lines arranged on the circumference of the mandril. It will be apparent that each of the labeled U-shaped bends on parallel reference lines in Figure 8 have been connected in Figures 10 and 11. For example, U-shaped bend 7' is shown to be connected to U-shaped bend 7 at the top of Figure 10. Although it is preferred that the U-shaped bends are welded, it is also possible to form the connecting portions of the filament from a single piece of material in order to eliminate the need for connecting each of the appropriate U-shaped bends. The ends of the filament are also connecting back to the filament and trimmed in order to remove any excess filament precluding from the free end.

The rigidity of the structure may be controlled by welding less than all of the adjacent curved portions. For example, a stent with only half the U-shaped portions welded together would be approximately half as rigid as a stent with all the tangent points welded together. Of course, the stent can also be used without any connections between adjacent curved portions.

The lowest possible profile (i.e. diameter) is provided by arranging the long leg of each U-shaped bend parallel to the axis of the catheter before it is inserted into a lumen. This arrangement increases the diameter to which the stent can be expanded without, at the same time, decreasing the end-to-end length of the stent. By increasing or decreasing the length of the long leg of each U-shaped bend, one can alter the expansion ratio without altering the profile. Consequently, a nearly unlimited circumferential expansion ratio may be created without contracting the stent along its longitudinal axis. The expansion ratio is therefore nearly independent of this profile.

When expanded, each of the U-shaped portions in the stent may assume a rhomboidal pattern where the legs of each U-shaped bend are no longer parallel. The expansion ratio of the stent may therefore exceed 10 to 1 in terms of the expanded diameter versus the unexpanded diameter of the stent. Consequently, the outside surface of the stent touching the vessel is small while the effective support area is very large. This feature dramatically reduces the possibility of causing any hemodynamic disturbances inside the vein or artery because of the stent. The large expansion ratio also allows the stent to be used with smaller incisions. Moreover, this configuration allows the stent to be flexible in the radial direction in order to accommodate the pulsation of an artery.

The stent may also be coated with anti-thrombolytic agents in order to limit the thrombotic formation which often accompanies angioplasty.

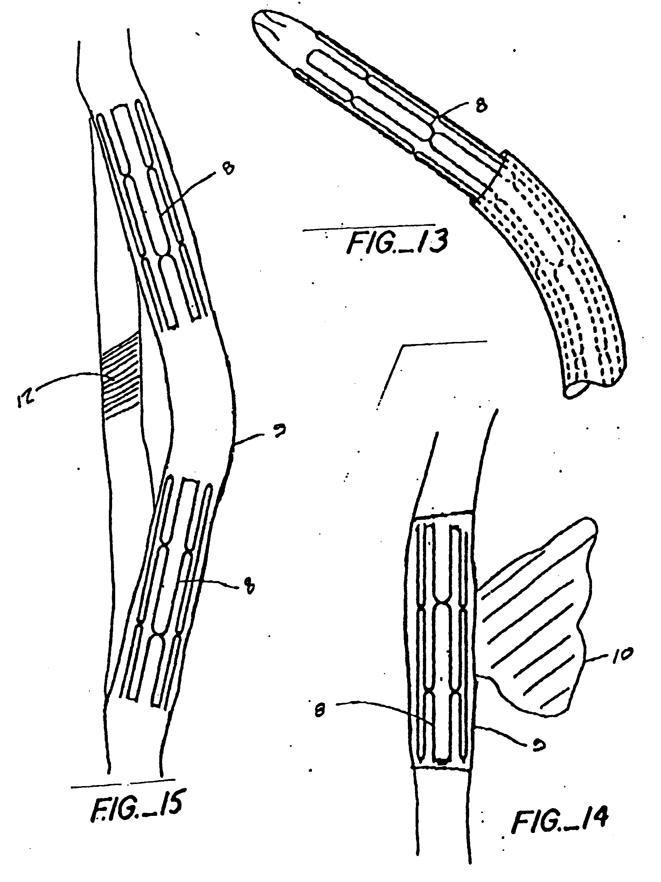

Figures 13-16 illustrate a typical stent of which could represent any one of the embodiments described above. Figure 13 shows a typical stent mounted on a 4/5 F balloon (4-10mm) with a 6/7 F sheath. The apparatus of Figure 13 is preferably used with a.078-.091 guide sheath. Figure 14 shows the stent, inside a graft, being used to repair a pseudo-aneurysm in a common femoral artery. The stent 8 is placed inside graft 9 which blocks off pseudo-aneurysm 13. Although the stent is shown to be completely inside graft 9, it may also extend outside the edges of the graft in order to provide additional support for the incisions at the end of the graft.

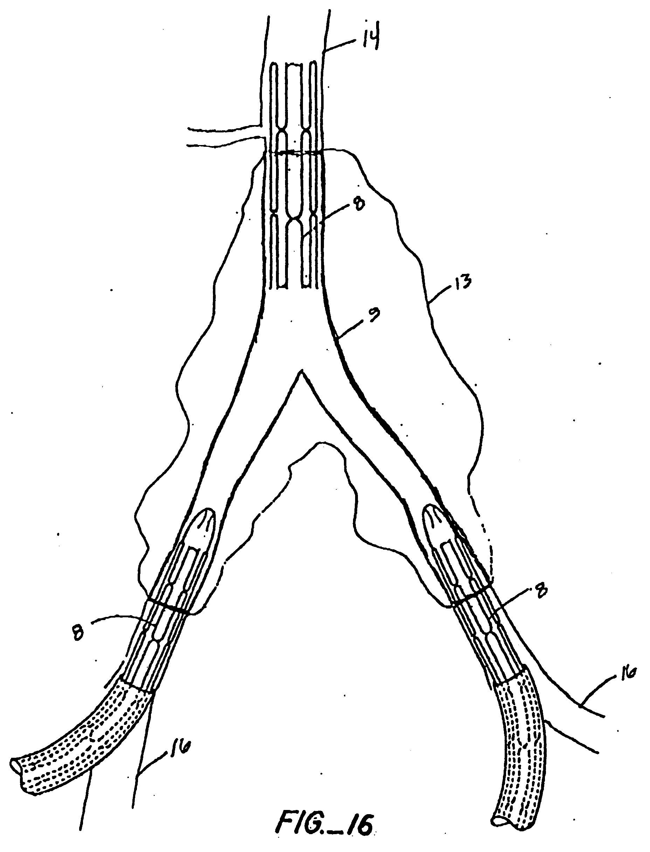

Figure 15 shows two stents being used at each end of a graft to bypass an occlusion 23 in, for example, the femoral-popliteal artery. Figure 16 illustrates how three stents can be used with a branched graft to repair an aorto-iliac aneurysm 33. The graft 9 is placed inside the aneurysm and secured at one end to the aorta 35. The other ends of the graft are similarly stented to iliac branches 37.

Figure 16 also illustrates how the catheter of Figure 17 can be used to insert the stent 8 inside a lumen. Typically, a short incision is made in the lumen (for example, a vein or artery) and the stent, which is mounted on the balloon, is then slipped into the incision. When the stent is in place, the balloon is expanded in order to expand the stent against the inside walls of the lumen. Once the stent is in place, the balloon is deflated and removed through the inside of the stent and the incision in order to leave the stent in place.

Various advantages of the present invention can now be understood. For example, the above-described stent uses substantially less material than conventional stents (especially knitted ones with overlapping wires) and, therefore, introduces a substantially lesser quantity of foreign material into a lumen. The stent also provides a maximum amount of structural support with a minimum amount of material. As another example, the above-described stent connects its filament ends back onto the lament to prevent thrombosis in blood vessels or damage to any type of a lumen wall such as is caused by stents that have loose wire ends that protrude into a lumen.

Another advantage of the above-described stent is that it provides substantial radial expansion with only limited longitudinal migration and, therefore, reduces the problem of migration inside a lumen. More particularly, the hoops and end component sections at each end of the above-described stent reduce migration by securing the stent inside of a lumen. In the preferred embodiment, the hoops, end component sections, as well as the spiral shape of the stent itself are oriented to inhibit longitudinal growth of the stent during radial expansion.

Yet another advantage of the above-described stent is that it provides sufficient flexibility to allow implantation in tortuous lumens and in applications where lumen bending is required. This overcomes the problem with conventional stents that are so stiff that they are difficult to negotiate through a tortuous vessel during implantation. Furthermore, a stiff stent can cause damage to certain vessels, such as those around joints, that require flexibility.

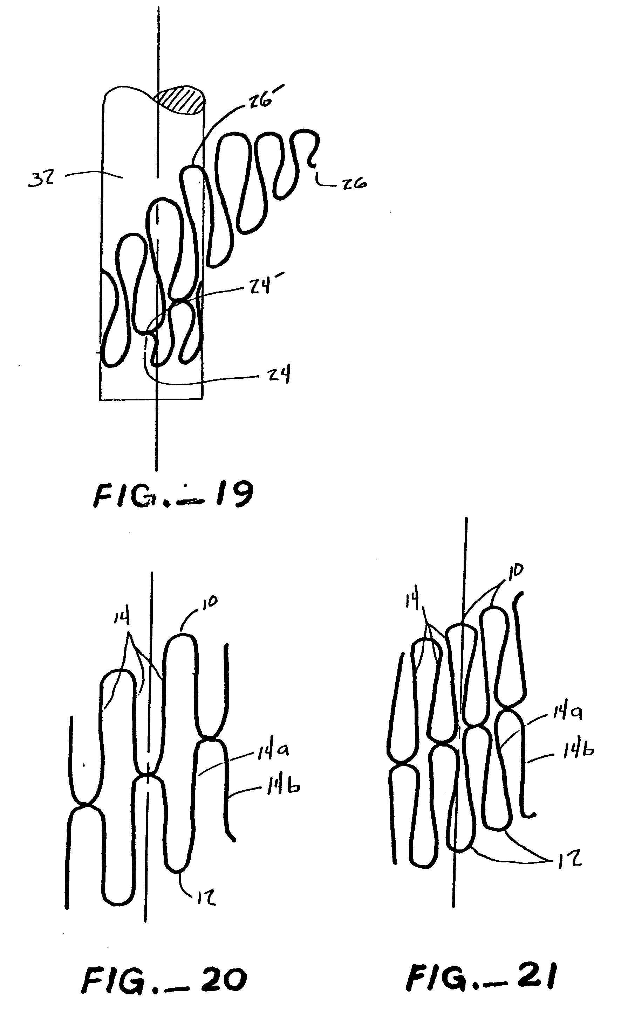

The stent is formed from a continuous wire shaped into the planar pattern or waveform illustrated in Figure 17. The pattern in Figure 17 includes a series of alternating U-shaped waves having a period p with peaks 10 and valleys 12 interconnected by substantially straight sections 14. The straight sections 14 are substantially parallel to each other in Figures 17, 18 and 20 and are therefore depicted as straight vertical lines in those figures. However, the term "substantially parallel" also refers to the configuration of the straight portions 14 illustrated in the compressed planar (and generally sinusoidal) waveforms of Figures 19 and 21. The peaks 10 and valleys 12 are preferably semicircular and arranged to intersect straight portions 14 at the tangent of each curved peak or valley so that there are no discontinuities in the wire. However, other curved or linear shapes may also be used to form the peaks 10 and valleys 12. Each U-shape wave includes an ascending side 14a and a descending side 14b.

The outermost portions of the peaks 10 and valleys 12 in the middle section of the waveform are aligned along parallel axes 16 and 18, respectively. The axes 16 and 18 form an acute angle α with respect to the straight portions 14. The angle α is preferably 45 degrees so that if distance between each straight section is one unit, then each U-shaped wave in the middle section has one leg that is three units long while the other leg is four units long as illustrated by the parallel horizontal reference lines in Figure 17. Other relative dimensions and angles, however, can be used. A curved stent can also be formed by, for example, slightly increasing the length of every third wave and decreasing the length of a corresponding wave in order to form an arched configuration where one side of the tubular body is slightly longer than another side.

There are two waves 20 of different amplitudes at each end of the stent which each have two sides of the same length. The end sections of the waveform include peaks 10a, 10b, and 10c at one end of the stent and valleys 12a, 12b, and 12c at the other end. The outer edges or apexes, of valleys 12a, 12b, and 12c are aligned along axis 28 which is substantially perpendicular to the straight portions 14 (i.e. horizontal in Figure 17). Similarly, the apexes of peaks 10a, 10b, 10c are aligned with an axis 30 which is also perpendicular to the straight portions 14 of the waves 22 but displaced from axis 30. The ends of the wire 24, 26 are preferably formed into half a valley 12 at one end and half of a peak 10 at the other end. The ends 26 may also include a small, straight portion (not shown) which may be parallel or perpendicular to the straight portions 14.

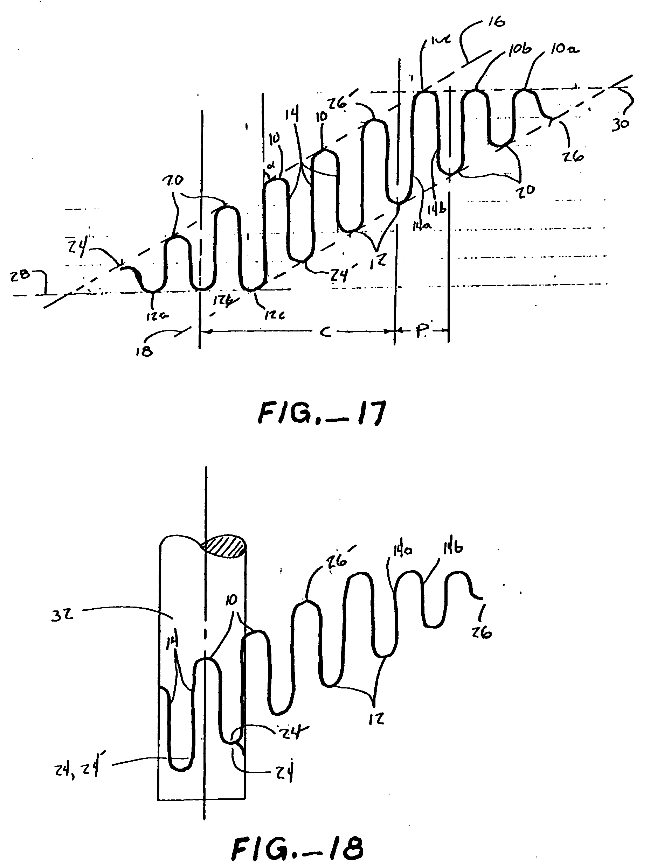

Referring to Figure 18, the stent is formed by wrapping the waveform of Figure 17 around a mandril 32. The peak 10 of one wave coincides with the valley 12 of another wave when the waveform of Figure 17 is wrapped around mandril 32 with straight portions 14 aligned with the longitudinal, or central, axis of the mandril 32. Figure 18 illustrates the end 24 of the waveform wrapped around the mandril 32 so that the end 24 is tangent to point 24'. Similarly, end 26 will be tangent to point 26' when the wave is completely wrapped around the mandril 32. The ends 24, 26 of some or all of the junctions are then bonded to one another over relatively short lengths to form bonded cells by spot welding, spot brazing, soldering, tying, looping, adhesive bonding, or other suitable means to the points 24' and 26' respectively, so that the ends of the wire are not exposed where they could snag or otherwise interfere with the placement of the stent in the vessel.

In practice, electric resistance welding has been found to offer the most secure metal to metal bond by minimizing the amount of oxidation that occurs during bonding process. As the wire is wrapped on the mandril, some or all of the successive junctions between the peaks 10 and valleys 12 may be bonded in a similar manner until the stent is complete. The flexibility of the stent can be controlled by bonding fewer than all of the peaks 10 to corresponding valleys 11.

The stent may then be compressed on consecutively small diameter mandrils so that the straight sections 14 in Figure 17 are no longer exactly parallel, but still "substantially parallel", i.e. less than 10° from being parallel, to the longitudinal axis of the mandril so the wave pattern takes on a generally sinusoidal shape such as the one illustrated in Figures 19 and 21. The planar waveform of Figure 17 may also be compressed perpendicular to straight sections 14 in order to form the nearly sinusoidal pattern illustrated in Figure 19 before being wrapped around the mandril 32. The stent is then removed from the smallest mandril and the stent is arranged on the balloon catheter.

The structure of the stent of the present invention is capable of expanding radially when subjected to the internal pressure of an expanding catheter balloon. The peaks 10 and valleys 12 between the waves operate like flexible junctions or hinges to allow the straight portions 14 to swing outwardly, oblique to the central axis of the body of the stent. Unlike hinges, however, after the stent is expanded, the junctions resist displacement of the straight sections in the opposite direction (for example, due to the compressive force of the lumen) which would tend to reduce the diameter of the expanded stent. The resistance of these junctions to compression (i.e. hoop strength) is caused by placing a stress on the material at the junction which exceeds the elastic limit of the material, so that the material near the junction is plastically deformed and thus resists any tendency for the stent to collapse inside a lumen. The wire and the bonding material should therefore be a low memory material.

Figures 17 and 18 illustrate a waveform where the period (or wavelength) of each wave p is roughly one-fourth of the mandril circumference c. This configuration has been found to minimize the number of waves, the number of bonds between waves, and amount of wire required to adequately support the lumen. For the embodiments illustrated in Figures 17 and 18, the end of the stent will have three peaks 10a, 10b and 10c, and three valleys 12a, 12b and 12c exposed on the end of the expanded stent. The apex of peaks 10a, 10b and 10c and valleys 12a, 12b and 12c are equally spaced at 120, 240 and 360 degrees, respectively, around the end face of the stent. This preferred configuration provides the maximum lumen support and minimum profile (i.e. diameter) in the unexpanded state using the least possible amount of foreign material inside the body. Conventional stents have been found to use more than three peaks or valleys around the end circumference of the body which increases their unexpanded profile and uses more material than is necessary. When the stent is properly expanded, each apex of peaks 10a-10c and valleys 12a-12c moves only in the radial direction away from the longitudinal axis of the tubular body of the stent. Consequently, the present stent will not migrate inside a lumen during expansion.

Figures 20 and 21 illustrate the arrangement of the waves (from the waveforms of Figures 18 and 19, respectively) around the circumference of the mandril 32 or body of the stent when the stent is in its unexpanded state. In both Figures 20 and 21, the straight portions 14 are "substantially parallel" to longitudinal axis of the tubular body of the stent which is illustrated by the centerline in each of the Figures.

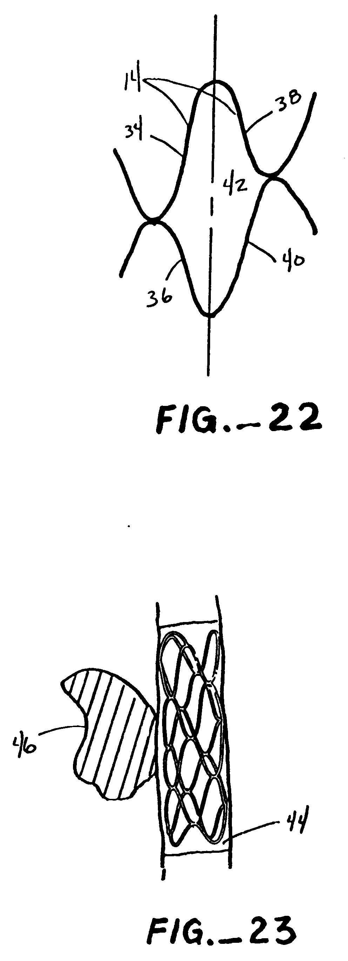

Figure 22 shows an enlargement of one of the cells 39 formed from the wave pattern of Figures 20 or 21 when the stent is in an expanded state. The cell 39 can also be described as a rhombic shape having four sides 34, 36, 38 and 40 where sides 34 and 36 are formed from one straight portion 14 and sides 38 and 40 are formed from another straight portion 14 which is adjacent to the other straight portion. The wire is bonded at the point of tangency between adjacent sides 34, 36 and 38, 40 of cell 39. It is clear from Figure 10, 11 and 22 that the straight portions will extend oblique to the central axis of the tubular body (shown by the centerline in the figures) when the stent is expanded to form a rhombic shaped cell.

The ultimate degree of expansion or expansion ratio of the stent can be adjusted by changing the height of the waves defined by the distance between axis 18 and axis 20. Increasing the length of straight sections 14 increases the ultimate expansion ratio of the stent without affecting its compressed or unexpanded diameter or profile. Consequently, the ultimate expanded diameter of the stent is independent of its unexpanded diameter so that one size stent can be used with almost any size lumen. Moreover, even large lumens can be supported with a stent that has a small unexpanded profile so that bleeding and vessel damage is minimized during implantation. In practice, the stent has been found to work well with expansion ratios of between 1:1 and 10:1; however, larger expansion ratios are also possible. The ultimate expansion ratio can also be increased by decreasing the period of the waves p and/or the distance between straight sections 14 so that more waves are created around the circumference of the stent.

Figure 23 shows the stent, inside a graft, being used to repair a pseudo-aneurysm in a common femoral artery. The stent 8 is placed inside graft 41 which blocks off pseudo-aneurysm 42. Although the stent is shown to be completely inside graft 41, it may also extend outside the edges of the graft in order to provide additional support for the incisions at the end of the graft.

In another preferred embodiment, a vascular prosthesis stent according to the present invention is constructed from a continuous wire that is half-round (i.e. semi-circular) in transverse cross-section. In other words, in transverse cross-section, the wire has a semi-circular side and a substantially planar side. In a completed stent, the semi-circular wire profiles are all on the exterior of the stent body while the planar portions of the wire are all on the interior. As a result, the interior of the stent -- comprised of the cross-sectional diameters of the wires --provides a generally smooth surface that minimizes blood flow turbulence along the interior of the stent.

As compared to full-round wire stents, the stent of this embodiment provides less topography or elevation of the stent in a vessel. This is important because the stent is a foreign body relative to the vessel and will elicit a tissue reaction that covers the stent and incorporates it into the vessel wall. In comparison to full-round wire stents, the stent of this embodiment reduces the thickness of foreign material which projects into the lumen and is in contact with flowing blood. Because the stent is generally flush with the vessel wall, it will incite a less exuberant, thinner layer of healing tissue to cover the prosthesis. This results in less compromise of the vessel lumen. Therefore, in comparison to full-round wire stents of the other embodiments, the stent of this embodiment will allow a larger luminal diameter than full-round wire stents and, therefore, provides a relatively larger internal flow diameter of blood flow through a vessel.

In this preferred embodiment, the vascular prosthesis stent has a sufficiently low profile (i.e. external diameter) in its compressed state that the stent can be inserted through a relatively small aperture in a blood vessel wall, thereby minimizing bleeding and damage to the vessel. Also, the low prole allows the stent to be easily moved through narrow vessels.

Further, the vascular prosthesis stent has a compressed profile which is independent of its expansion ratio. In other words, the ultimate expanded diameter of the stent is not a function of its compressed profile and, therefore, one size stent can be used for lumens of a wide range of diameters.

Still further in this preferred embodiment, the vascular prosthesis stent has substantial flexibility in its compressed state while being generally rigid and having a high hoop strength in its expanded state. The flexibility of the compressed stent is important, as mentioned above, for inserting the stent through tortuous lumens. The hoop strength is important for resisting the radial forces from the artery after the stent is in place. Also, with the stent being substantially rigid after it is expanded inside a vessel, movement of the stent against the vessel intima is reduced after the stent is implanted. The reduction in movement is important for reducing trauma and for promoting healing of the vessel.

Even further still, the vascular prosthesis stent of this embodiment, has a tubularly-shaped body comprised of a plurality of oblong, open cells which are staggered around the circumference of the body such that when the stent is in its compressed condition, the long sides of each oblong cell are substantially parallel to the stent's longitudinal axis. The adjoining cells normally are bonded together at a point between adjacent parallel sides on a cell so that, when the stent is expanded, the adjacent sides of each cell extend at an oblique angle to the longitudinal axis of the stent.

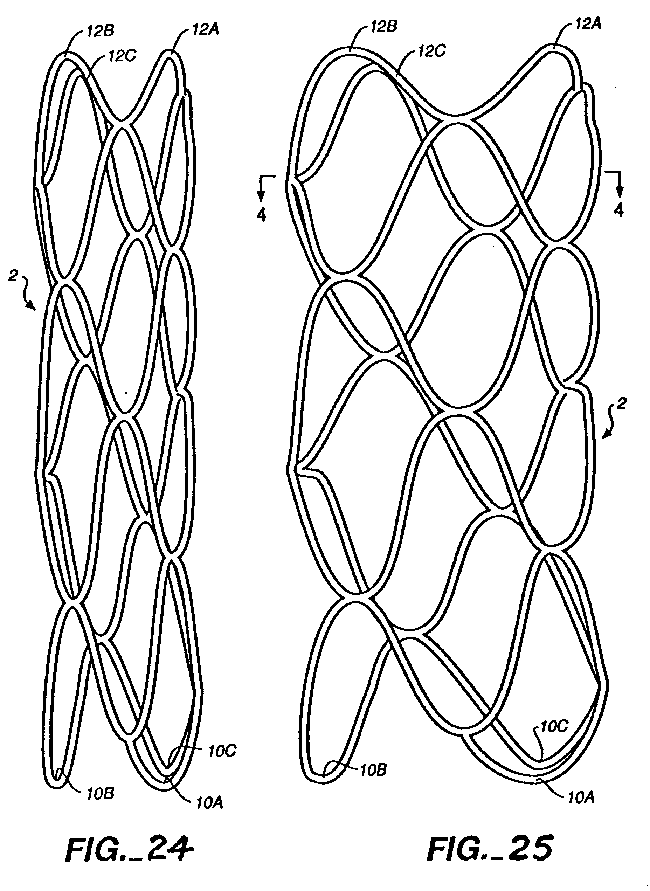

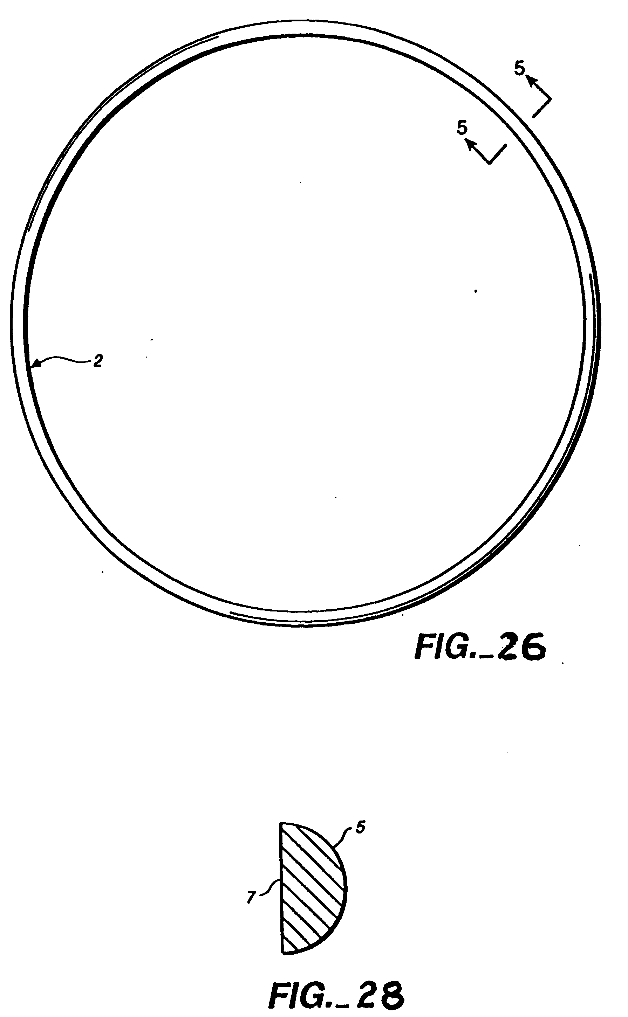

A vascular prosthesis stent, as shown in Figures 24 to 26, has a tubularly-shaped body 22 formed from a continuous wire or the like. The tubularly-shaped body preferably is comprised of a plurality of cells that are formed from the continuous wire, with each of the cells having a plurality of sides. The cell sides extend substantially parallel to the longitudinal axis of the tubularly-shaped body when it is compressed (Figure 24), but extend obliquely to the longitudinal axis of the tubularly-shaped body when it is expanded (Figure 25). The construction of the stent is as described above except that, as can be seen in Figure 28, the continuous wire that forms the tubularly-shaped stent body is half-round (i.e., semi-circular) in transverse cross-section. In other words, in transverse cross-section, the wire has a semi-circular side 25 and a substantially planar side 27. The substantially planar side 27 generally corresponds to the diameter of the wire. In practice, the planar side is smooth and has a polished appearance.

From the following, it can be understood that it is important for the stent wire to have a substantially planar side, but it is not necessary that the remainder of the periphery of the wire be semi-circular. Indeed, the remainder of the periphery of the wire can have a variety of arcuate and non-arcuate shapes.

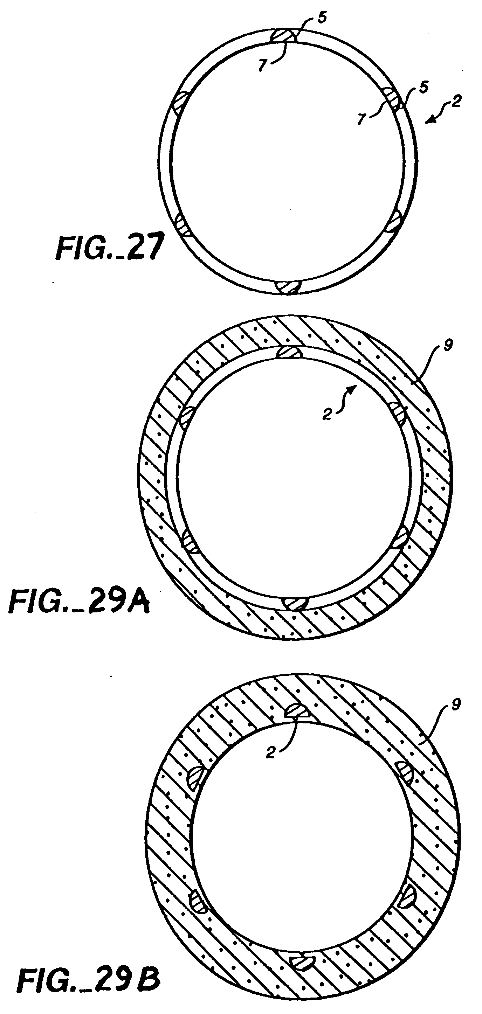

As can be seen in Figure 27, the continuous wire is wound such that the semi-circular wire profiles 25 are all on the exterior of the tubularly-shaped stent body while the planar portions 27 are all on the interior of the stent. As compared to a full-round wire design, the orientation of the half-round wire is important so that the interior of the stent -- comprised of the cross-sectional diameters of the wires --provides a generally smooth surface that minimizes blood flow turbulence along the interior of the stent and reduces the thickness of reactive tissue required to cover the prosthesis and incorporate it into vessel wall.

In use of the above-described stent, the stent is maneuvered along a blood vessel until it reaches desired location, whereat the stent is expanded by a balloon catheter for lodging inside of a lumen. When so expanded, the semi-circular profiles of the wires on the exterior of the stent press into the vessel wall. In fact, as suggested by Figure 29A, the stent may expand sufficiently that all of the semi-circular profiles on the exterior of the stent are embedded in a vessel wall 29 to the extent that the planar portions of the wire are substantially flush with the vessel wall. As a result, the interior of the lumen is generally smooth without impedance from the embedded stent.

There are several benefits to the stent to the configuration shown in Figure 24. One benefit, as mentioned above, is that the stent offers a generally smooth surface that reduces turbulence on blood flowing along the lumina supported by the stent and encourages blood platelet aggregation. As a result, this configuration minimizes the traumatic effect of the stent on vessels and blood cells. Further, this configuration promotes healing of the vessel.

As compared to full-round wire stents, the stent of this embodiment provides less topography, or elevation, of the stent in the vessel. This is important because the stent configuration allows its planar surface to be embedded in a manner substantially flush with the inner surface of the vessel wall. Consequently, the normal healing reaction of the vessel wall in response to the stent insertion is relatively thin and less exuberant than that required to incorporate a full-round wire design which projects further into the lumen from the vessel wall. As an example, Figure 29B shows the vessel of Figure 29A with tissue healed over the stent; typically, the tissue layer (intimal hyperplasia) is about 100 angstroms thick.

Also in comparison to full-round wire stents, the stent of this embodiment requires less reactive tissue to incorporate the stent into the vessel wall. Again this is important because the neointimal layer will be completed faster when the reaction requires less reactive tissue. Finally in comparision to full-round wire stents, because the stent of this embodiment elicits a thinner circumferential layer of tissue having, it can yield a larger luminal diameter than full-round the stents and, therefore, provides a larger internal flow diameter for blood flow.

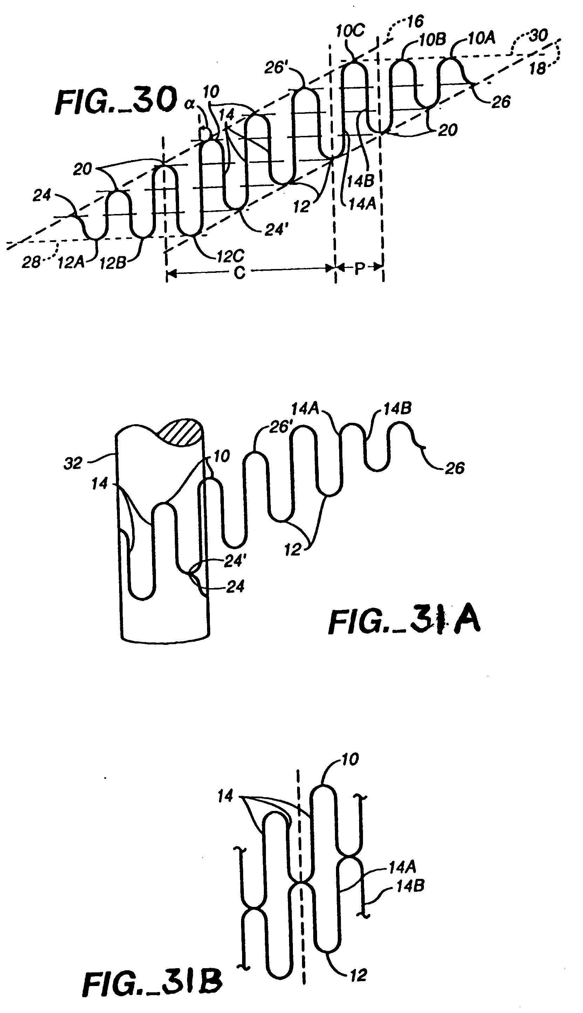

Figures 30 and 31 A show patterns or waveforms of the wire that forms the stent similar to that described in connection with Figures 17 and 18.

As further shown in Figure 30, the peaks 10 and valleys 12 are interconnected by substantially straight sections 14. The straight sections 14 are substantially parallel to each other and, for that reason, are depicted as straight vertical lines in the drawings. (The term "substantially parallel" is intended to encompass the configuration of the straight portions 14 in the compressed and expanded stent.) In practice, the peaks and valleys are generally semicircular in shape and arranged to intersect the straight portions 14 at the tangent of each curved peak or valley, with the result that there are no discontunuities in the wire.

The formation of the stent about the mandrel in the other preferred embodiment can be summarized by observing that the continuous wire is formed into an asymmetric undulating wave pattern around the circumference of the tubularly-shaped stent body with each wave having a long ascending side and a short descending side, with a peak between the long ascending side and the short descending side valley between the short descending side and a long ascending side of an adjacent wave, and the ascending and descending sides of each wave being arranged substantially parallel to the longitudinal axis of the body when the body is in compressed condition. Further, as the continuous wire is wound around the cylindrical mandril, the wire configuration is adjusted so that the n^{th} peak comes into tangency with the valley immediately following peak n^{th}+3, and so forth so that all peaks and valleys are in tangency. Then pairs of the tangent points are fixed together over relatively short lengths by means of for example a spot weld to form a plurality of cells arranged substantially parallel to the long axis of the mandrel. Preferably, the long side of the wave and the short side of the wave are in a ratio of about 4:3. Also, as mentioned above, at least some of the peaks and valley of the waves are bonded together to form a plurality of cells.

It should be particularly noted the waveform is wrapped around the mandrel 32 so that the planar face of the half-round wire is in contact with the mandrel. That is, the mandrel surface is tangent to the substantially planar face of the half-round wire and the semi-circular surface of the half-round wire faces outward from the mandrel. Thus, when the tubularly-shaped stent is removed from the mandrel, it is in the compressed condition shown in Figure 24.

It should also be noted that the end 24 of the waveform is wrapped around the mandril 32 so it is tangent to point 24'. Similarly, end 26 is tangent to point 26' when the wave is completely wrapped around the mandril 32. In practice, the ends 24, 26 are bonded (as by welding, brazing, soldering, tying. looping, adhesive bonding, or other suitable means) so that the ends of the wire are not exposed to snag or otherwise interfere with the placement of the stent in the vessel.

The planar waveform is compressed perpendicular to straight sections 14 to form an undulating pattern before being wrapped around the mandril 32. In these conditions, the straight portions 14 are substantially parallel to longitudinal axis of the tubularly-shaped stent body.

Referring again to Figure 24, it can be seen that the side profile of the stent in its expanded state is defined by cells that have generally rhombic shapes with four sides. As mentioned above, the wire is bonded at the tangent points between adjacent sides to form bonded cells. The above-discussed straight portions 14 extend obliquely to the central axis of the tubularly-shaped body when the stent is expanded as shown in Figure 25.

In operation, the compressed stent is mounted on a catheter for insertion into a lumen. Then, during implantation, the compressed stent 22 and a catheter balloon are withdrawn inside the sheath onto the catheter while the sheath is slid inside a vessel lumen. Then, after the compressed stent 22 is moved to its appropriate position, the sheath is partially withdrawn so that the compressed stent 22 and the balloon are exposed inside the lumen. The balloon is then inflated and the stent 22 is expanded inside the lumen. Finally, the balloon is deflated and the catheter is removed from the lumen without the stent.

The stent material preferably has "low memory," which is to say that it does not try to resume its original shape after it is deformed. This is important for preventing the stent from recoiling to its compressed condition after implantation. In one preferred embodiment, the stent is formed from about (0.006 to 0.020 inch) 0.015 to 0.050 diameter annealed tantalum wire. The stent material may also be radio-opaque to allow its location in a vessel to be verified through fluoroscopic examination. Preferably, the stent is made from a biocompatible material (such as stainless steel) or a bio-absorbable material (such as Vicryl). The stent may also be coated with anti-thrombolytic or anti-coagulant agents such as "Dextran", "Heperin", (R.T.M's) t-PA, polytetrafluoroethylene, or ultra low-temperature isotropic carbon.

It is important for the stent wire to have a substantially planar side, but the remainder of the periphery of the wire can have a variety of arcuate and non-arcuate shapes.

It has been found by the present inventor that an ideal vascular prosthesis should include several features. The stent should be formed from as little material as possible with a low profile (i.e. diameter) in its unexpanded state so that it can be inserted through the smallest possible hole in the vessel wall in order to control bleeding and damage to the vessel. A low profile also allows the stent to be more easily moved through narrow vessels. Furthermore, it is preferable that the unexpanded profile of the stent be independent of its expansion ratio. In other words, besides needing the smallest possible profile during insertion, there is also a need to be able to change the ultimate expansion ratio of the stent without affecting its unexpanded profile so that one size stent can be used with almost any size lumen.

The stent should also have high flexibility in its unexpanded state and excellent hoop strength in its expanded state. In practice, it has been found to be difficult to design a stent with both of these characteristics. Flexibility is needed to insert the stent through tortuous lumens while hoop strength is needed to resist the radial forces from the artery once the stent is in place. The stent should also be rigid once it is expanded inside a vessel in order to minimize its movement against the vessel intima after it is in place and to promote healing of the vessel after placement. Furthermore, the flexibility of the design should be adjustable without changing the size or configuration of the stent.

The stent should be altraumatic to vessels and blood cells. It should therefore be formed from as little biocompatible material as possible. The stent should not have any exterior tines or sharp edges which could damage the wall of the vessel. It should also not have any interior tines which could damage the catheter balloon or cause hemodynamic disturbances which might interfere with the flow of blood through the stent. The material from which the stent is formed is preferably a low memory, radio-opaque material. In other words, the stent should maintain its shape without recoil once it is expanded inside the vessel and should be visible during fluoroscopy in order to be able to verify that the stent has not migrated from its intended position.

In the preferred embodiments, the vascular stent includes a continuous wire which is formed into a substantially tubular body. The wire forms a plurality of oblong, open cells which are staggered around the circumference of the tube. When the body is formed in its unexpanded state, the long sides of each oblong cell are arranged substantially parallel to the longitudinal or axis of the tubular body. Adjoining cells may then be bonded together at a point between adjacent parallel sides on a cell. When the body is expanded, the adjacent sides of each cell extend oblique to the longitudinal axis of the body.

## Claims

1. A vascular stent (8) comprising a tubular body with a plurality of cells each cell having a plurality of sides, the sides (14) of the cells extending substantially parallel to a longitudinal axis of the tubular body when the latter is in an unexpanded state, and the sides (14) of at least certain of the cells extending obliquely to the longitudinal axis when the tubular body is in an expanded state, the sides of each cell being made of wire with adjacent cells having wire in common, characterised in that adjacent cells are fixed together by two bonded wires (1,1'), one from each cell, said two bonded wires being in a non-overlapping and abutting relationship at the position of a bend in the wire.

2. A stent according to claim 1, wherein the plurality of cells are formed from a continuous wire, and wherein the said two bonded wires are two parts of said continuous wire.

3. A stent according to claim 1 or 2, wherein upon expansion of the stent each common wire is bent in the region of the two bonded wires and is formed as rhomboid shaped cells.

4. A stent according to claim 3, wherein the bent wire is deformed past its elastic limit whereby the expanded tube will retain its expanded state.

5. A stent according to any one preceding claim, wherein the adjacent sides of said two bonded wires have been spot welded together.

6. A stent according to any one preceding claim, wherein the wire is of substantially semicircular cross section with the flat side thereof inward facing.

7. A method of making a vascular stent, said stent comprising a tubular body with a plurality of cells each cell having a plurality of sides, the sides (14) of the cells extending substantially parallel to a longitudinal axis of the tubular body when the latter is in an unexpanded state, and the sides (14) of at least certain of the cells extending obliquely to the longitudinal axis when the tubular body is in an expanded state, wherein the sides of each cell being made of wire with adjacent cells having wire in common, and adjacent cells being fixed together by two bonded wires (1,1'), one from each cell, said two bonded wires being in a non-overlapping and abutting relationship at the position of a bend in the wire and wherein the plurality of cells are formed from a continuous wire, the said two bonded wires being two parts of said continuous wire, characterized in that the method comprises the steps of forming the wire into a substantially sinusoidal wave pattern, and wrapping the wire around a mandril to form the stent, the said wave pattern having substantially straight portions which are substantially aligned with the longitudinal axis of the mandrel, and in that the wire has a semicircular cross-section with the flat portion facing the interior of the stent.

8. A method according to claim 7, further CHARACTERISED BY the step of coupling or bonding together at discrete locations on adjacent sides of adjacent cells so that the stent is formed into a plurality of interconnected cells each of which can rotate about the said discrete locations to form a rhomboid shape upon expansion of the stent.

9. A method according to claim 8, CHARACTERISED IN THAT the substantially sinusoidally shaped wire is substantially U-shaped arranged so that upon winding longitudinally spaced valleys coincide with longitudinally spaced peaks, and in that the peaks and valleys are coupled or fixed together, and in that substantially straight adjacent cells sides of the U-shaped wires are fixed together at specific locations about which the wire rotates upon expansion to form the rhomboid shaped cells.

## Patentansprüche

1. Gefäß-Stent (8) mit einem rohrförmigen Körper mit einer Mehrzahl von Zellen, die jeweils eine Mehrzahl von Seiten aufweisen, wobei die Seiten (14) der Zellen im wesentlichen parallel zu einer Längsachse des rohrförmigen Körpers verlaufen, wenn sich dieser in einem ungedehnten Zustand befindet, und die Seiten (14) mindestens bestimmter der Zellen schräg zur Längsachse verlaufen, wenn sich der rohrförmige Körper in einem gedehnten Zustand befindet, wobei die Seiten jeder Zelle aus Draht gefertigt sind und benachbarte Zellen Draht gemeinsam haben, dadurch gekennzeichnet, daß benachbarte Zellen durch zwei verbundene Drähte (1, 1'), einen von jeder Zelle, aneinander befestigt sind und die beiden verbundenen Drähte sich nicht überlappen, sondern an der Stelle einer Biegung im Draht aneinanderstoßen.

2. Stent nach Anspruch 1, bei dem die Mehrzahl von Zellen aus einem durchgehenden Draht gebildet ist und bei dem die beiden verbundenen Drähte zwei Teile des durchgehenden Drahtes sind.

3. Stent nach Anspruch 1 oder 2, bei dem während der Dehnung des Stents jeder gemeinsame Draht im Bereich der beiden verbundenen Drähte gebogen und zu rhomboidartigen Zellen geformt wird.

4. Stent nach Anspruch 3, bei dem der gebogene Draht über seine Elastizitätsgrenze hinaus verformt wird, wodurch das gedehnte Rohr seinen gedehnten Zustand beibehält.

5. Stent nach einem der vorhergehenden Ansprüche, bei dem die benachbarten Seiten der beiden verbundenen Drähte mittels Punktschweißung miteinander verschweißt sind.

6. Stent nach einem der vorhergehenden Ansprüche, bei dem der Draht einen im wesentlichen halbkreisförmigen Querschnitt hat, dessen flache Seite nach innen weist.

7. Verfahren zum Herstellen eines Gefäß-Stents mit einem rohrförmigen Körper mit einer Mehrzahl von Zellen, die jeweils eine Mehrzahl von Seiten aufweisen, wobei die Seiten (14) der Zellen im wesentlichen parallel zu einer Längsachse des rohrförmigen Körpers verlaufen, wenn sich dieser in einem ungedehnten Zustand befindet, und die Seiten (14) mindestens bestimmter der Zellen schräg zur Längsachse verlaufen, wenn sich der rohrförmige Körper in einem gedehnten Zustand befindet, wobei die Seiten jeder Zelle aus Draht gefertigt sind, benachbarte Zellen Draht gemeinsam haben und benachbarte Zellen durch zwei verbundene Drähte (1, 1'), einen von jeder Zelle, aneinander befestigt sind und die beiden verbundenen Drähte sich nicht überlappen, sondern an der Stelle einer Biegung im Draht aneinanderstoßen, und wobei die Mehrzahl von Zellen aus einem durchgehenden Draht gebildet sind und die beiden verbundenen Drähte zwei Teile des durchgehenden Drahtes sind, dadurch gekennzeichnet, daß man dem Draht eine im wesentlichen sinusförmige Wellenform verleiht und den Draht zur Bildung eines Stents um einen Dorn wickelt, wobei die Wellenform im wesentlichen gerade Anschnitte aufweist, die im wesentlichen auf die Längsachse des Dorns ausgerichtet sind, und daß der Draht einen im wesentlichen halbkreisförmigen Querschnitt hat, dessen flache Seite zum Innern des Stents weist.

8. Verfahren nach Anspruch 7, weiterhin dadurch gekennzeichnet, daß man an bestimmten Stellen an benachbarten Seiten benachbarter Zellen ein Aneinanderkoppeln oder Verbinden vornimmt, so daß der Stent in eine Mehrzahl von untereinander verbundenen Zellen geformt wird, von denen sich jede so um die bestimmten Stellen drehen kann, daß sie bei Dehnung des Stents eine rhomboidartige Form annimmt.

9. Verfahren nach Anspruch 8, dadurch gekennzeichnet, daß der im wesentlichen sinusförmig geformte Draht im wesentlichen so in U-Form angeordnet ist, daß beim Wickeln in Längsrichtung beabstandete Täler mit beabstandeten Spitzen zusammenfallen, und daß die Spitzen und Täler aneinander gekoppelt oder befestigt werden und daß im wesentlichen gerade benachbarte Zellenseiten der U-förmigen Drähte an bestimmten Stellen aneinander befestigt werden, um die sich der Draht beim Dehnen dreht, so daß die rhomboidartig geformten Zellen gebildet werden.

## Revendications

1. Stent vasculaire (8) comprenant un corps tubulaire avec une pluralité de cellules, chaque cellule ayant une pluralité de côtés, les côtés (14) des cellules s'étendant essentiellement parallèlement à un axe longitudinal du corps tubulaire lorsque ce dernier est dans un état non déployé, et les côtés (14) d'au moins certaines des cellules s'étendant obliquement par rapport à l'axe longitudinal lorsque le corps tubulaire est dans un état déployé, les côtés de chaque cellule étant fabriqués en fil métallique, les cellules adjacentes ayant du fil métallique en commun, caractérisé en ce que les cellules adjacentes sont fixées les unes aux autres par deux fils métalliques liés (1, 1'), un provenant de chaque cellule, lesdits deux fils métalliques liés étant dans une relation de non chevauchement et d'aboutement au niveau de la position d'une courbure dans le fil métallique.

2. Stent selon la revendication 1, dans lequel la pluralité de cellules est formée à partir d'un fil métallique continu, et dans lequel lesdits deux fils métalliques liés sont deux parties dudit fil continu.

3. Stent selon la revendication 1 ou 2, dans lequel lors du déploiement du stent, chaque fil métallique commun est courbé dans la région des deux fils métalliques liés et est formé en tant que cellules de forme rhomboïdale.

4. Stent selon la revendication 3, dans lequel le fil métallique courbé est déformé au-delà de sa limite élastique, ce par quoi le tube déployé conservera son état déployé.

5. Stent selon l'une quelconque des revendications précédentes, dans lequel les côtés adjacents desdits deux fils métalliques liés ont été soudés par point ensemble.

6. Stent selon l'une quelconque des revendications précédentes, dans lequel le fil métallique a une section transversale essentiellement semicirculaire avec son côté plat tourné vers l'intérieur.

7. Procédé de fabrication d'un stent vasculaire, ledit stent comprenant un corps tubulaire avec une pluralité de cellules, chaque cellule ayant une pluralité de côtés, les côtés (14) des cellules s'étendant essentiellement parallèlement à un axe longitudinal du corps tubulaire lorsque ce dernier est dans un état non déployé, et les côtés (14) d'au moins certaines des cellules s'étendant obliquement par rapport à l'axe longitudinal lorsque le corps tubulaire est dans un état déployé, dans lequel les côtés de chaque cellule sont fabriqués en fil métallique, les cellules adjacentes ayant du fil métallique en commun, et les cellules adjacentes étant fixées les unes aux autres par deux fils métalliques liés (1, 1'), un provenant de chaque cellule, lesdits deux fils métalliques liés étant dans une relation de non chevauchement et d'aboutement au niveau de la position d'une courbure dans le fil métallique, et dans lequel la pluralité de cellules est formée à partir d'un fil métallique continu, lesdits deux fils métalliques liés étant deux parties dudit fil métallique continu, caractérisé en ce que ce procédé comprend les étapes consistant à former le fil métallique suivant un motif d'onde essentiellement sinusoïdale, et à enrouler le fil métallique autour d'un mandrin pour former le stent, ledit motif d'onde ayant essentiellement des portions droites qui sont essentiellement alignées avec l'axe longitudinal du mandrin, et en ce que le fil métallique a une section transversale semicirculaire, la portion plate étant tournée vers l'intérieur du stent.

8. Procédé selon la revendication 7, caractérisé en outre par l'étape consistant à coupler ou à lier ensemble en des emplacements discrets sur des côtés adjacents des cellules adjacentes, de sorte que le stent soit formé suivant une pluralité de cellules interconnectées, chacune pouvant tourner autour desdits emplacements discrets pour former une forme rhomboïdale lors du déploiement du stent.

9. Procédé selon la revendication 8, caractérisé en ce que le fil métallique de forme essentiellement sinusoïdale est arrangé essentiellement en forme de U de sorte que lors de l'enroulement, des vallées espacées longitudinalement coïncident avec des pics espacés longitudinalement, et en ce que les pics et les vallées sont couplés ou fixés ensemble, et en ce que les côtés de cellules adjacentes essentiellement droits des fils métalliques en forme de U sont fixés les uns aux autres en des emplacements spécifiques autour desquels le fil métallique tourne lors du déploiement pour former les cellules de forme rhomboïdale.
